# EUROPEAN PATENT APPLICATION

(11) **EP 4 524 579 A1**
(43) Date of publication of application: **19.03.2025**
(21) Application number: 23803622.2
(22) Date of filing: 12.05.2023
(51) Int. Cl.: G01N 35/08, C12M 1/40, C12N 11/14, G01N 27/28, G01N 27/327, G01N 27/416, G01N 37/00

(54) **FLUID DEVICE**

(30) Priority: 13.05.2022 JP 2022079253
(71) Applicant: Provigate Inc., Tokyo 113-0033 (JP)
(72) Inventor: MIYAZAWA, Yuya, Tokyo 113-0033 (JP); KUMAGAI, Yoko, Tokyo 113-0033 (JP); ITO, Narushi, Tokyo 113-0033 (JP)
(74) Representative: Michalski Hüttermann & Partner Patentanwälte mbB
(86) International application number: PCT/JP2023/017822
(87) International publication number: WO 2023/219148

(57) **Abstract**

The present disclosure provides a fluidic device including a device main body having a disk-shaped space for containing a fluid; a fluid inlet configured to introduce the fluid tangentially into the disk-shaped space at the 0 o'clock position of a substantially circumferential portion; and a fluid outlet configured to guide the fluid out of the disk-shaped space at the 6 o'clock to 12 o'clock position of the substantially circumferential portion of the space.

## Description

### TECHNICAL FIELD

The present disclosure relates to a fluidic device.

### BACKGROUND ART

Microfluidic devices with small volumes have been developed to measure small-volume liquid samples.

When a fluid is introduced into a flow channel device, voids or dead spaces are likely to occur in the corners of the space. Due to resistance from the walls, a target solution is introduced into a relatively central portion of the volume, and the previous liquid may remain near the walls. This results in uneven solution distribution in the fluidic device. The influence of these problems on the measurement results is small as long as the volume of the fluidic device is large. Measurements can be performed in areas where no problems occur. That is, these problems can be ignored.

### SUMMARY OF INVENTION

However, as the flow channel device is smaller, the generation or remaining of air voids or uneven distribution of the target solution increases with respect to the area or volume measured, and the behavior of the fluid in the microspace is more pronounced. Therefore, these problems can substantially affect the results or efficiency of microfluidic measurements. Such a problem is merely an example, and the problem of the present disclosure described below is not limited thereto.

It is desired to improve the generation or remaining of voids and fluid exchange efficiency in a microfluidic device. The present disclosure describes and provides a microfluidic device that reduces or avoids these and other problems.

The present disclosure provides a fluidic device including:
a device main body having a disk-shaped space for containing a fluid;
a fluid inlet configured to introduce the fluid in a clockwise and tangential direction into the disk-shaped space at the 0 o'clock position of a substantially circumferential portion of the disk-shaped space; and
a fluid outlet configured to guide the fluid out of the disk-shaped space at the 6 o'clock to 12 o'clock position of the substantially circumferential portion of the space.

According to some embodiments of the present disclosure, for example, highly accurate measurements can be efficiently performed on a relatively small amount of a sample.

Additional aspects and advantages of the present disclosure will become readily apparent to those skilled in the art from the following detailed description, wherein only exemplary embodiments of the present disclosure are shown and described. As will be realized, the present disclosure is capable of other and different embodiments, and its several details are capable of modifications in various obvious respects, all without departing from the disclosure. Accordingly, the drawings and description are to be regarded as illustrative in nature, and not as restrictive.

### BRIEF DESCRIPTION OF DRAWINGS

[Fig. 1] Fig. 1 is a schematic top view illustrating a fluidic device according to a comparative example.
[Fig. 2] Fig. 2 is a schematic top view illustrating a fluidic device according to a comparative example.
[Fig. 3] Fig. 3 illustrates schematic top views of fluidic devices according to some embodiments.
[Fig. 4] Fig. 4 illustrates schematic top views of fluidic devices according to some embodiments.
[Fig. 5] Fig. 5 illustrates a schematic top view (A) and a schematic perspective view (B) of a fluidic device according to an embodiment.
[Fig. 6] Fig. 6 illustrates a schematic top view (A) and a schematic perspective view (B) of a fluidic device according to an embodiment.
[Fig. 7] Fig. 7 is a schematic top view illustrating a fluidic device according to an embodiment.
[Fig. 8] Fig. 8 is an exploded perspective view illustrating a fluidic device according to an embodiment.
[Fig. 9] Fig. 9 is a schematic top view illustrating a fluidic device according to a comparative example.
[Fig. 10] Fig. 10 is a graph illustrating the output versus the amount introduced in a fluidic device according to an embodiment and a fluidic device according to a comparative example.
[Fig. 11] Fig. 11 is a graph illustrating a change in output over time at each height of a disk-shaped space according to an example.
[Fig. 12] Fig. 12 is a graph illustrating the relationship between the height of the disk-shaped space and the duration of maximum output in an example.

### DESCRIPTION OF EMBODIMENTS

The term "disk-shaped space" used in this specification typically refers to a cylindrical space. The size (major and/or minor axis size, diameter, or radius) of a cross section of the cylinder is greater than or equal to the size in the direction of the central axis. In some embodiments, the cylinder of the "disk-shaped space" has a substantially perfect circular cross section. Its diameter or radius is greater than the size in the direction of the central axis (the height of the disk-shaped space).

An "inlet for fluid"(also referred to as a "fluid inlet" or "inlet") and/or an "outlet for fluid" (also referred to as a "fluid outlet" or "outlet") has an internal tubular space (flow channel) and may be detachably connected to a device main body, may be fixed to the device main body, or may be integrally formed or manufactured with the device main body. The inlet and/or outlet may also refer to the flow channel itself.

The term "0 o'clock" used in the present specification refers to the angular position of the disk space where a fluid flowing from the fluid inlet has substantially entered the disk space. The direction from the center of the disk space toward the connection position of the fluid inlet to the disk space may also be defined as 0 o'clock. Zero o'clock may also be defined as the radial direction perpendicular to the circumferential direction at the time of fluid introduction. The introduced fluid or at least a portion thereof flows circumferentially near the circumference of the disk-shaped space. **In** the present specification, this flow direction is defined as clockwise.

The inlet and/or outlet are disposed at, substantially at, or near a circumferential portion. The term "circumferential portion" used in the present specification refers to at or near the circumference of the disk of the disk-shaped space of the fluidic device. This does not refer to a location on the geometric circumference, but rather to a position or location that is at least as far away from the geometric circumference as is necessary to introduce or withdraw fluid into or from the disk-shaped space. For example, the inlet and/or outlet has a tubular structure. At least part or the whole of the diameter of the tubular structure is contained in the disk. **In** this case, the center of the inlet or outlet tube does not lie on the circumference of the disk. **In** some cases, due to manufacturing reasons, the inlet and/or outlet cannot be formed on the circumference or cannot be formed in such a manner that the outer edge thereof is exactly on the circumference.

The term "disk-shaped space" refers to a space that is formed in a cylindrical shape. In some embodiments, a section perpendicular to the central axis of the cylinder may be a perfect circle. In some embodiments, the section of the cylinder may be non-circular, for example, elliptical. The circumferential surface of the disk-shaped space may be formed as a substantially curved surface, preferably a continuous curved surface.

In some embodiments, the fluid inlet may be configured to introduce a fluid in a direction perpendicular to the central axis of the cylinder of the disk-shaped space, i.e., in an in-plane direction of the disk. In some embodiments, the connection may be made to the side surface of the disk from outside the disk-shaped space.

In some embodiments, the fluid inlet may be configured to introduce a fluid in a direction perpendicular to the central axis of the cylinder of the disk-shaped space, i.e., in a direction inclined from the in-plane direction of the disk. In some aspects, the fluid inlet may be connected to a base of the cylinder. In some embodiments, the fluid inlet may be connected to the base of the cylinder in a direction that is non-perpendicular or inclined to the base of the cylinder. That is, the direction of the fluid inlet projected onto the base of the cylinder points in the circumferential direction of the cylinder.

In some embodiments, the inclined inlet and outlet may be connected to the same base of the disk-shaped space. In some embodiments, the inclined inlet may be connected to a first base (one base), and the outlet may be connected to a second base (the other base).

In some embodiments, the two bases need not necessarily be parallel. For example, each base need not be flat. For example, at least a part thereof may be formed in a conical shape (convex direction or concave direction with respect to the disk space). For example, one base of the cylinder may be configured to be spaced apart from the other base near the fluid inlet (the convex direction cone). This allows, for example, any air bubbles in the disk space to more easily exit from the fluid outlet. For example, one base of the cylinder may be configured to be close to the other base near the fluid inlet (the concave direction cone). A fluid introduced into the disk space is likely to pass near the circumference. A water flow on the circumferential side with a relatively small solution exchange rate can be intensified to increase the solution exchange rate.

The height of the disk-shaped space may be a value, such as 10 µm, 15 µm, 20 µm, 25 µm, 30 µm, 40 µm, 50 µm, 60 µm, 70 µm, 80 µm, 90 µm, 100 µm, 150 µm, 200 µm, 300 µm, 400 µm, 500 µm, 600 µm, 700 µm, 800 µm, 900 µm, or 1,000 µm, or a value greater than that.

The height of the disk-shaped space may be a value, such as 10 µm, 15 µm, 20 µm, 25 µm, 30 µm, 40 µm, 50 µm, 60 µm, 70 µm, 80 µm, 90 µm, 100 µm, 150 µm, 200 µm, 300 µm, 400 µm, 500 µm, 600 µm, 700 µm, 800 µm, 900 µm, 1,000 µm, 1.5 mm, or 2 mm, or a value smaller than that.

The radius or the feature quantity in the surface direction of the disk-shaped space may be a value, such as 1 mm, 2 mm, 3 mm, 4 mm, 5 mm, 6 mm, 7 mm, 8 mm, 9 mm, or 10 mm, or a value greater than that.

The radius or the feature quantity in the surface direction of the disk-shaped space may be a value, such as 5 mm, 6 mm, 7 mm, 8 mm, 9 mm, 10 mm, 11 mm, 12 mm, 13 mm, 14 mm, 15 mm, 16 mm, 17 mm, 18 mm, 19 mm, or 20 mm, or a value smaller than that.

The volume of the disk-shaped space may be a value, such as 1 µL, 2 µL, 3 µL, 4 µL, 5 µL, 6 µL, 7 µL, 8 µL, 9 µL, 10 µL, 15 µL, 20 µL, 30 µL, 40 µL, 50 µL, 60 µL, 70 µL, 80 µL, 90 µL, 100 µL, 200 µL, 300 µL, 400 µL, or 500 µL, or a value greater than that.

The volume of the disk-shaped space may be a value, such as 5 µL, 6 µL, 7 µL, 8 µL, 9 µL, 10 µL, 15 µL, 20 µL, 30 µL, 40 µL, 50 µL, 60 µL, 70 µL, 80 µL, 90 µL, 100 µL, 200 µL, 300 µL, 400 µL, 500 µL, or 1 mL, or a value smaller than that.

The fluid inlet may have a volume that is substantially 50% to 200% of the volume of the disk-shaped space. The volume of the fluid inlet may be substantially 50%, 100%, 150%, or 200% of the volume of the disk-shaped space. The sum of the volume of the disk-shaped space and the volume of the inlet (volume from the fluid introduction portion to the inlet to the disk-shaped space) may be a value, such as 2 µL, 3 µL, 4 µL, 5 µL, 6 µL, 7 µL, 8 µL, 9 µL, or 10 µL, or a value greater than that. The sum of the volume of the disk-shaped space and the volume of the inlet may be a value, such as 10 µL, 15 µL, 20 µL, 30 µL, 40 µL, or 50 µL, or a value smaller than that.

In some embodiments, the fluidic device may have a sensor at an inner wall of the disk-shaped space. In some embodiments, the sensor may be disposed on one or both of the base inner walls of the disk-shaped space.

### <Measurement Target>

In some embodiments, a target (subject) may include or may be a human. In some embodiments, the target may include an animal other than a human or may be an animal other than a human. The target may include a mammal or may be a mammal. Non-limiting examples of the target include a working animal, a domestic animal, a pet animal, and a wild animal.

The sample to be measured may be a solution. The "solution" may be a body fluid, a solution derived from a body fluid, or a dilute fluid of a body fluid. The solution may be a solution that is not a body fluid (derived from a non-body fluid), or may be a mixture of a body fluid or a solution derived from a body fluid and a solution derived from a non-body fluid. The solution may be a solution used for a sample measurement or a solution used for a calibration measurement. For example, the solution may be a standard solution or a calibration solution. For example, the solution may be a liquid intentionally or deliberately free from a target substance to be measured, as it is used for calibration or the like. The sample to be measured may be a specimen. The solution may be a solution containing a chemical substance.

The "body fluid" may be lymph fluid, may be tissue fluid, such as interstitial fluid, intercellular fluid, or interstitial fluid, body cavity fluid, serosal cavity fluid, pleural fluid, ascites fluid, pericardial fluid, cerebrospinal fluid (spinal fluid), joint fluid (synovial fluid), and aqueous humor of the eye (aqueous). The body fluid may be a digestive juice, such as saliva, gastric juice, bile, pancreatic juice, or intestinal juice, and may be sweat, tears, nasal mucus, urine, semen, vaginal fluid, amniotic fluid, or milk. The body fluid may be a body fluid of an animal or may be a body fluid of a human. The "body fluid" may be a solution. The solution may contain a physiological buffer, such as phosphate-buffered saline (PBS) or an N-tris(hydroxymethyl)methyl-2-aminoethanesulfonic acid buffer solution (TES), containing a substance to be measured. The solution is not particularly limited as long as it contains the substance to be measured.

The solution may contain a target substance to be measured. The solution may have the possibility to contain the target substance to be measured. In some embodiments, the target substance to be measured may be, for example, a molecule, an ion, a macromolecule, or a biomolecule. The target substance to be measured may contain a biomolecule. The target substance to be measured may be, for example, a protein or a glycated protein. For example, the solution is tears, and the target substance to be measured may be albumin, glycoalbumin, hemoglobin, or glycohemoglobin contained in tears. Alternatively, the target substance to be measured may be albumin, glycoalbumin, hemoglobin, or glycohemoglobin in blood, serum, or plasma, or may be albumin, glycoalbumin, hemoglobin, or glycohemoglobin in interstitial fluid, urine, or saliva. The albumin may be oxidized albumin (HNA) or reduced albumin (HMA). In some embodiments, the target substance to be measured may be AGE (an advanced glycation end products, late-stage glycation products). In some embodiments, the target substance to be measured may be a glycated lipid.

### <Sensor>

A sensor may be, for example, a chemical sensor, a biosensor, or an ion sensor (hereinafter sometimes referred to as a "sensor", a "biochemical sensor", a "chemical sensor", or an "electrochemical sensor"). The sensor may include multiple sensors.

The sensor may include an electrode. The electrode may be an amperometric electrode. The electrode may include a hydrogen peroxide electrode. The electrode may include an oxygen electrode. The electrode may be a potentiometric electrode. The electrode may be an electrode for ion detection (for example, a pH electrode, a cyanide ion electrode, or an iodide ion electrode).

In some embodiments, the sensor may output an electrical signal. In some embodiments, the sensor may output a current signal. The sensor may output a voltage signal or an electric charge. The sensor may be electrically coupled to, for example, an ammeter or a voltmeter.

**In** some embodiments, the sensor may have an enzyme membrane over the electrode.

**In** some embodiments, the enzyme membrane may contain a protease. "Protease" is a generic term for peptide-bond hydrolases that hydrolyze and catabolize proteins and polypeptides. A protease may be an enzyme that breaks down a protein into peptide fragments. When a protein contains a glycated amino acid residue, peptide fragments produced by the action of a protease may include peptide fragments containing a glycated amino acid residue and peptide fragments that are not glycated at all.

The "protease" may be a protease derived from an animal, a protease derived from a plant, or a protease derived from a microorganism. The protease may be an exopeptidase or an endopeptidase. The protease may be an aspartic protease, a metalloprotease, a serine protease, or a thiol protease.

The "protease" may include multiple types or kinds of protease, and may include one type or kind of protease. For example, the protease may contain one or both of a proteinase and a peptidase. Mixing multiple proteases may increase the efficiency of degradation.
The protease may contain a modification-type protease or a modified protease. The proteases may be used with an additive. The additive may be, for example, a surfactant or urea. The additive can destabilize or denature proteins, for example without limitation. By use of a modified protease or an additive, the efficiency of degradation of a protein and the selectivity of the base material can be improved, for example without limitation.

A "substrate" is a substance that catalyzes a chemical reaction by an enzyme. Alternatively, a substrate is a substance that binds to an enzymatic protein, undergoes a decrease in the activation energy of a particular chemical reaction upon binding to an enzyme, and, as a result, is converted to a particular product at an amazing rate.

**In** some embodiments, the enzyme membrane may contain an oxidase. "Oxidases" (oxidizing enzymes) are enzymes that use a substrate of molecular oxygen as an electron acceptor. Alternatively, oxidases are enzymes that catalyze redox reactions involving oxygen molecules as acceptors for hydrogen or electrons.

**In** some embodiments, the oxidase may contain a ketoamine oxidase. **In** some embodiments, the oxidase may contain a glucose oxidase. This can be used to measure the glucose concentration in a sample. In that case, the substrate may contain glucose. In some embodiments, the oxidase may contain an alcohol oxidase. In that case, the substrate may contain a primary alcohol. This can be used to measure the alcohol concentration in a sample.

The term "ketoamine oxidase" generally refers to an oxidase that recognizes a ketoamine structure of a glycated amino acid or a peptide or peptide fragment containing a glycated amino acid residue and oxidizes the glycated amino acid to produce an amino acid, glucosone (α-ketoaldehyde), and hydrogen peroxide. Thus, ketoamine oxidase generates hydrogen peroxide in a concentration proportional to or related to the concentration of the peptide or peptide fragment containing the glycated amino acid or glycated amino acid residue to be recognized.

The ketoamine oxidase may be a dehydrogenase, a kinase, or an oxidase. The ketoamine oxidase may be fructosyl amino acid oxidase (FAOD), fructosyl peptide oxidase, fructosyl valyl histidine oxidase, fructosyl amine oxidase, amadoriase, fructosyl amine deglycase, or a modified form thereof.

In some embodiments, the sensor can measure a glycated protein. In some embodiments, a protease and a ketoamine oxidase may be disposed on or near the surface of the electrode. In some embodiments, the enzyme membrane may contain a protease and a ketoamine oxidase. A typical method for measuring a glycated protein using an enzymatic method is as follows: In a first step, a protein is degraded into amino acids using protease. In a second step, only a glycated amino acid of these amino acids is reacted with a ketoamine oxidase to generate hydrogen peroxide. In the third step, the hydrogen peroxide is optically or electrically measured.

In some embodiments, the sensor may include a detection portion. The detection portion may be a hydrogen peroxide detection portion. The "hydrogen peroxide detection portion" (hydrogen peroxide sensor) may be an electrode of an electrochemical type or a hydrogen peroxide electrode. The hydrogen peroxide electrode may have a counter electrode, a reference electrode, and a working electrode. In an embodiment, the detection portion may detect oxygen. For example, the amount or concentration of oxygen that decreases in an enzymatic reaction may be detected. Oxygen detection is considered to be relatively insensitive to molecules and ions as noise sources and to be resistant to interference. Oxygen consumption may be measured by oxygen detection. The detection portion is atmospherically saturated and thus may be used for sensing an enzyme. The detection portion may be configured in such a manner that multiple detection methods can be used selectively or in combination.

### <Optical Measurement>

In some embodiments, the main body of the fluidic device may be configured to guide light from the outside into the interior of the disk-shaped space. The main body of the fluidic device may be at least partially transparent.

The fluidic device may include an optical device or may be configured to be connected to an optical device.

The term "optical measurement" used in the present specification typically refers to the determination of optical properties of a substance using an optical element or device. In some embodiments, optical measurement of a target substance may be measured. In some embodiments, the properties of a substance bound to or associated with a target substance (hereinafter, a substance (for example, a reagent) chemically, biologically, or physically bound to or associated with the target substance, even if it is not the target substance itself) may be measured. Properties of the reagent may be measured. The reagent may be referred to as a "target substance".

In some embodiments, the optical measurement may include a spectroscopic measurement. For example, the absorbance of the target substance may be measured. In some embodiments, a color indicator corresponding to the target substance may be introduced. The color indicator may be detected or measured.

The fluidic device may include multiple disk-shaped spaces. Each disk may have an inlet and an outlet. A fluid may be provided to multiple inlets and disk-shaped spaces from a common flow channel. A fluid may be provided to each of the multiple inlets and disk-shaped spaces individually. The multiple disk-shaped spaces included in the fluidic device may have substantially the same volume, or may have different volumes from each other.

In some embodiments, the fluidic device may be a stopped-flow fluidic device. After the disk-shaped space is filled with the introduced fluid, the introduction of fluid may be stopped. Thereafter, predetermined measurements or sensing may be performed on the fluid in the disk-shaped space.

In some embodiments, a predetermined measurement or sensing may be performed on the fluid in the disk-shaped space while the introduced fluid is flowing through the disk-shaped space.

### <Comparative Example 1>

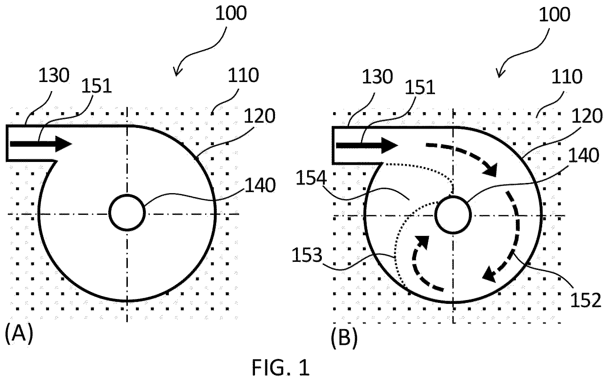

Fig. 1A is a schematic view of a fluidic device 100 as a comparative example. The fluidic device 100 has a disk-shaped space 120 formed in a main body 110. The fluid inlet 130 and the fluid outlet 140 are fluidly connected to this disk-shaped space 120. The fluid inlet 130 in Fig. 1A is disposed in a cross-sectional direction. A fluid 151 (solid arrow) that has passed through the fluid inlet 130 is introduced into the disk-shaped space 120 perpendicularly to the 0 o'clock direction, that is, in the circumferential direction. The fluid outlet 140 in Fig. 1A is disposed almost at the center of the disk-shaped space 120 and guides the internal fluid to the outside of the disk-shaped space 120 from the base (not illustrated).

Fig. 1A illustrates a section perpendicular to the central axis of this disk-shaped space 120. The fluid outlet 140 is actually connected to either base of the disk-shaped space 120 and is not present on the cross section, but is illustrated in the same figure for illustrative purposes. The same applies to the following figures.

Fig. 1B illustrates the same fluidic device 100 as in Fig. 1A and is used to explain an example of the flow of the fluid 151 in the disk-shaped space 120. The fluid 151 enters the disk-shaped space 120 through the fluid inlet 130 and then flows through its circumferential portion (see dashed lines 152). However, the fluid outlet 140 is in the central portion of the disk-shaped space 120. Thus, the fluid 152 flowing through the circumferential portion does not go around the circumferential portion, but leaves the circumferential portion at the position approximately between 6 o'clock and 9 o'clock, for example. The fluid 152 then flows toward the fluid outlet 140 or is drawn to the fluid outlet 140 at the center of the disk-shaped space 120. Thus, a liquid surface 153 is formed, and a space 154 (sometimes referred to as an "air bubble") where no liquid is present is formed, with the liquid surface serving as the boundary. As a result, the disk-shaped space 120, especially its base, cannot be completely filled with the fluid.

### <Comparative Example 2>

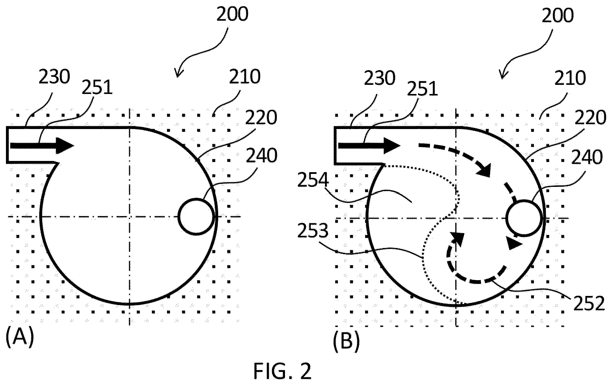

Fig. 2A is a schematic view of a fluidic device 200 as a comparative example. The fluidic device 200 has a disk-shaped space 220 formed in a main body 210. A fluid inlet 230 and a fluid outlet 240 are fluidly connected to the disk-shaped space 220. The fluid inlet 230 in Fig. 2A is disposed in a cross-sectional direction. A fluid 251 (solid arrow) that has passed through the fluid inlet 230 is introduced into the disk-shaped space 220 perpendicularly to the 0 o'clock direction, that is, in the circumferential direction. The fluid outlet 240 in Fig. 2A is disposed near the circumference of the disk-shaped space 220 at the 3 o'clock position, and guides the internal fluid to the outside of the disk-shaped space 220 from the base (not illustrated).

Fig. 2B illustrates the same fluidic device 200 as in Fig. 2A and is used to explain an example of the flow of the fluid 251 in the disk-shaped space 220. The fluid 251 enters the disk-shaped space 220 through the fluid inlet 230 and then flows through its circumferential portion (see dashed lines 252). The fluid outlet 240 is at the 3 o'clock position of the disk-shaped space 220. A portion of the fluid 252 that has flowed through the circumferential portion exits from the fluid outlet 240, but the rest continues to flow along the circumferential portion. The fluid 252 does not go around the circumferential portion, but leaves the circumferential portion at the position approximately between 6 o'clock and 9 o'clock, for example. The fluid 252 then flows toward the fluid outlet 240 or is drawn to the fluid outlet 240. Thus, a liquid surface 253 is formed, and a space 254 (sometimes referred to as an "air bubble") where no liquid is present is formed, with the liquid surface serving as the boundary. As a result, the disk-shaped space 220, especially its base, cannot be completely filled with the fluid.

The fluids 152 and 252 (flows indicated by dashed lines) in the disk-shaped spaces 120 and 220 illustrated in Figs. 1 and 2 are illustrative and should not be construed as limiting. Other fluid flows are also possible. In some embodiments, the disk-shaped spaces 120 and 220 may be completely filled with the introduced fluids.

### <Configuration of Fluidic Device>

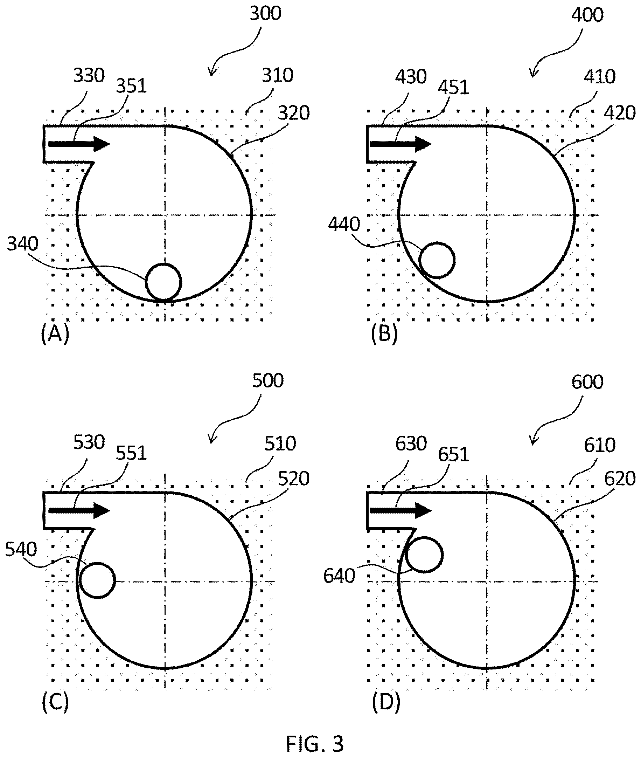

Figs. 3A to 3D illustrate configurations of fluidic devices according to some embodiments. Fig. 3A illustrates a fluidic device 300 with a fluid outlet 340 disposed near the circumference of a disk-shaped space 320 at the 6 o'clock position. Fig. 3B illustrates a fluidic device 400 with a fluid outlet 440 disposed near the circumference of a disk-shaped space 420 at approximately the 7:30 o'clock position. Fig. 3C illustrates a fluidic device 500 with a fluid outlet 540 disposed near the circumference of a disk-shaped space 520 at the 9 o'clock position. In general, air bubbles are less likely to remain in the disk-shaped space when the fluid outlet is located closer to the fluid inlet.

Fig. 3D shows a fluidic device 600 with a fluid outlet 640 disposed near the circumference of a disk-shaped space 620 at approximately the 10 o'clock position. The outlet 640 in Fig. 3D is disposed as close to a fluid inlet 630 as possible without interfering with the fluid inlet 630. At this position, the fluid introduced from the fluid inlet 630 and the fluid flowing along the circumferential portion of the disk-shaped space 620 meet, easily generating turbulence. Therefore, air (space or an air bubble where no liquid is present) remains easily. The fluid outlet 640 can efficiently vent the air bubble seen in Figs. 1B and 2B to the outside.

As illustrated in Figs. 1 to 3, the cross section of each of the disk-shaped spaces may be a circle. Alternatively, the cross section of each disk-shaped space need not be a circle, may be other curved lines, or may be composed of multiple curved surfaces. In some embodiments, the cross section of the disk-shaped space may be an ellipse.

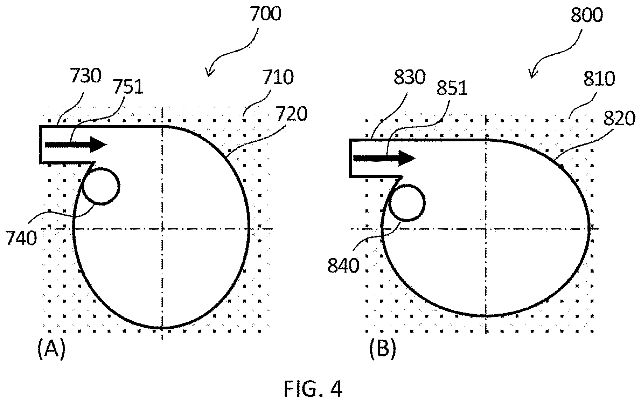

Figs. 4A and 4B illustrate fluidic devices 700 and 800 having elliptical disk-shaped spaces. The fluidic device 700 illustrated in Fig. 4A has the major axis of an ellipse in the 0 o'clock-6 o'clock direction of a disk-shaped space 720. A fluid 751 is introduced into the disk-shaped space 720 in the circumferential direction at the 0 o'clock position, which is at one end of the major axis, through a fluid inlet 730. The fluidic device 800 illustrated in Fig. 4B has the major axis of an ellipse in the 3 o'clock-9 o'clock direction of a disk-shaped space 820. A fluid 851 is introduced into the disk-shaped space 820 in the circumferential direction at the 0 o'clock position, which is at one end of the minor axis, through the fluid inlet 830. In both cases, the fluid outlets 740 and 840 are disposed at approximately 10 o'clock or in a location close to but not interfering with the fluid inlets 730 and 830.

In some embodiments, the fluid inlet may be disposed in the in-plane direction of the cross-section of the disk-shaped space. In some embodiments, the fluid inlet may be disposed at an angle to the cross section of the disk-shaped space.

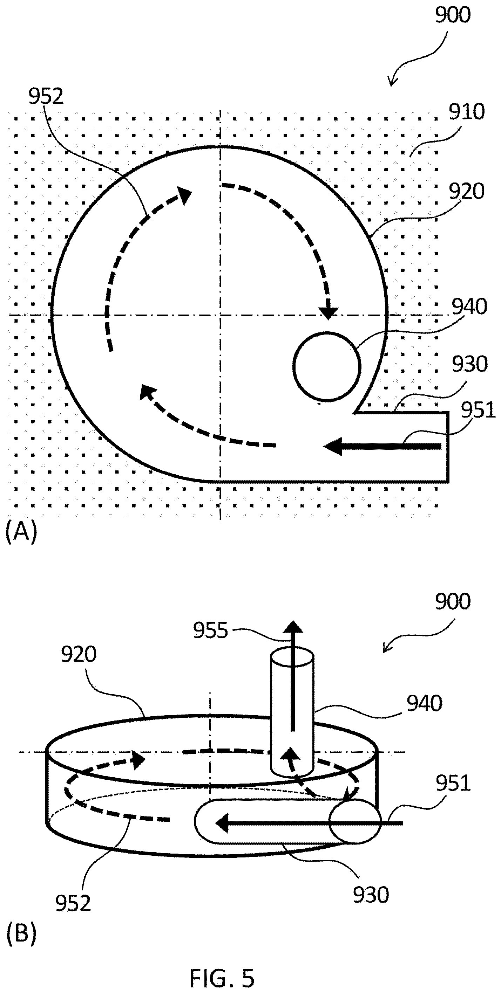

Fig. 5 illustrates a fluidic device 900 in an embodiment, in which a fluid inlet is disposed in the in-plane direction of a cross section of a disk-shaped space. Fig. 5A illustrates a section, or a top view, parallel to the base of the fluidic device 900. Fig. 5B is a perspective view of the fluidic device 900 with a main body 910 omitted. The fluidic device 900 has a fluid inlet 930 disposed in an in-plane direction parallel to the base of a disk-shaped space 920.

The fluid inlet 930 introduces a fluid 951 in the in-plane direction into the disk-shaped space 920 at the 0 o'clock position (actually, a position close to 11 o'clock to the 0 o'clock (12 o'clock) position). The fluid (dashed line 952) flows mainly along the circumference in the disk-shaped space 920 and fills the disk-shaped space 920. The fluid 952 finally exits from a fluid outlet 940 disposed substantially perpendicular to one base of the disk-shaped space 920.

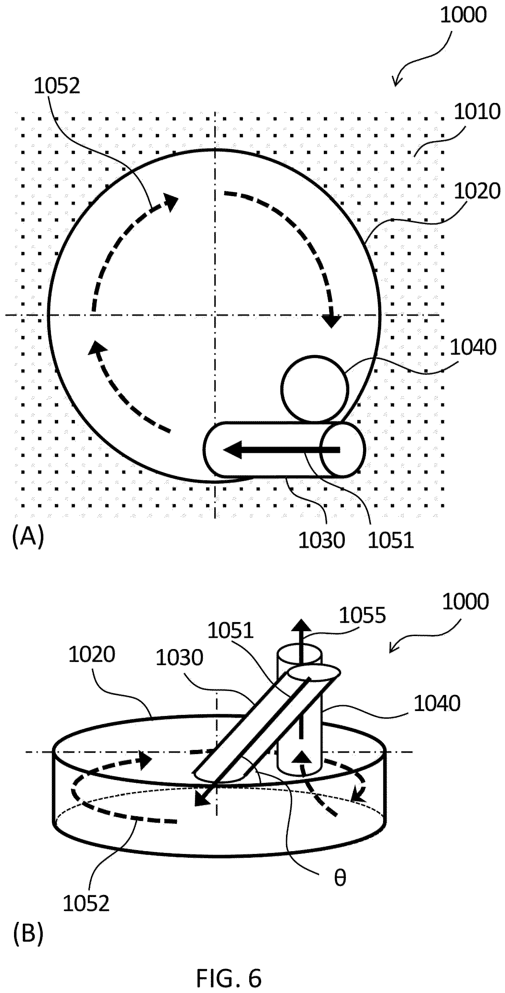

Fig. 6 illustrates a fluidic device 1000 according to an embodiment, in which a fluid inlet is disposed at an angle to a cross section of a disk-shaped space. Fig. 6A illustrates a cross section parallel to the base, or a top view, of the fluidic device 1000. Fig. 6B is a perspective view of the fluidic device 1000 with the main body 1010 omitted. The fluidic device 1000 has a fluid inlet 1030 disposed at an angle θ to the base of a disk-shaped space 1020.

The fluid inlet 1030 introduces a fluid 1051 into the disk-shaped space 1020 in the direction of an angle θ at the 0 o'clock position of the disk-shaped space 1020 (actually, from a position close to 11 o'clock to the 0 o'clock (12 o'clock) position). The fluid (dashed line 1052) flows mainly along the circumference in the disk-shaped space 1020 and fills the disk-shaped space 1020. The fluid 1052 finally exits from a fluid outlet 1040 disposed substantially perpendicular to one base of the disk-shaped space 920.

### <Fluidic Device with Sensor - 1>

In some embodiments, a fluidic device may include a sensor in its disk-shaped space. The sensor can sense a fluid introduced into the disk-shaped space or a substance contained in the fluid.

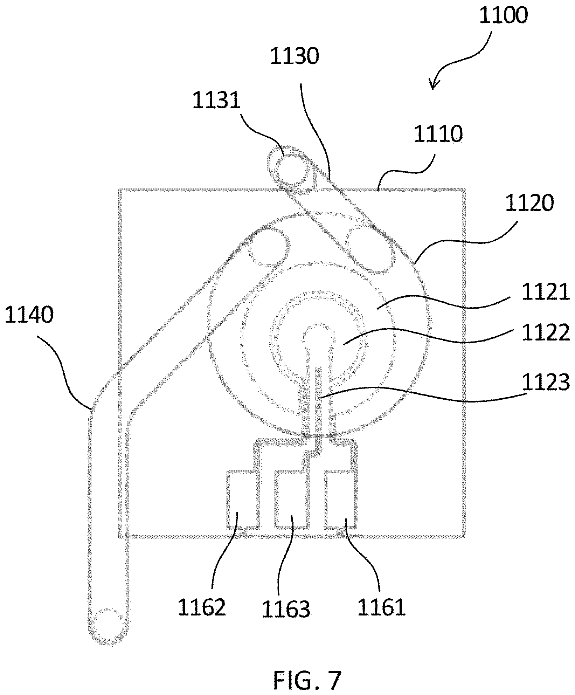

Fig. 7 illustrates a top view of a fluidic device 1100 according to an embodiment. However, the solid and dashed lines are not to be construed as corresponding to whether or not they are visible from the top, but are merely used to schematically illustrate the configuration of components in a fluidic device 1100.

The fluidic device 1100 has a disk-shaped space 1120 inside its main body 1110. A fluid inlet 1130 and a fluid outlet 1140 are arranged for the disk-shaped space 1120. The fluid inlet 1130 is disposed at an angle to the base of the disk-shaped space 1120. The fluid outlet 1140 is disposed perpendicular to the base of the disk-shaped space 1120. A fluid introduction port 1131 is disposed perpendicular to the plane of the main body 1110 or the disk-shaped space 1120, and introduces a fluid introduced from the outside into a fluid introduction port 1131. A fluid then enters the fluid inlet 1130 and is introduced obliquely into the interior of the disk-shaped space 1120. The fluid outlet 1140 is disposed perpendicular to the plane of the main body 1110 or the disk-shaped space 1120. The fluid is guided from the disk-shaped space 1120 to the outside through the fluid outlet 1140.

In the disk-shaped space 1120, a sensing electrode including electrodes 1121, 1122, and 1123, is disposed. In this embodiment, the sensing electrode includes three electrodes for electrochemical measurement. The sensing electrode includes a working electrode 1121, a counter electrode 1122, and a reference electrode 1123. These electrodes are connected to respective connection terminals 1161, 1162, and 1163 with lead wires provided therebetween. The connection terminals are exposed on the outer surface of the main body 1110 and can be electrically connected from the outside.

### <Fluidic Device with Sensor - 2>

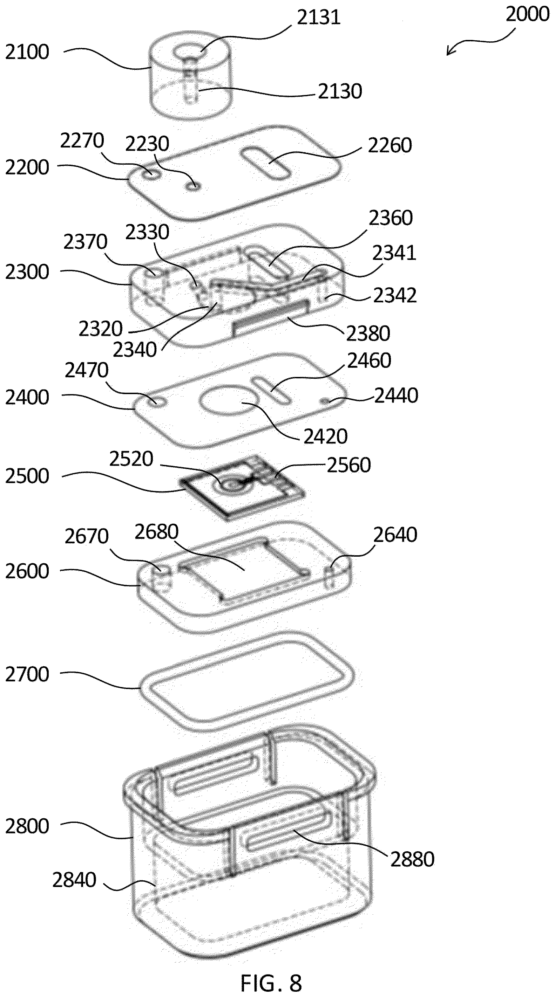

Fig. 8 illustrates an exploded perspective view of a fluidic device 2000 according to an embodiment. The fluidic device 2000 includes, from top to bottom, a pipette port 2100, a top seal 2200, a flow channel cell 2300, an adhesive film 2400, a sensor chip 2500, a sensor chip support 2600, an O-ring 2700, and a waste liquid tank 2800, which are assembled together in the vertical direction.

The sensor chip support 2600 has a sensor chip receiving portion 2680 in which the sensor chip 2500 is placed. The flow channel cell 2300 is firmly attached to the top of this with the adhesive film 2400 provided therebetween.

The flow channel cell 2300 has a disk-shaped depression 2320 on its lower surface. This disk-shaped depression 2320, a surface of an electrode section 2520, facing the disk-shaped depression, on the upper surface of the sensor chip 2500, and the through-hole 2320 of the adhesive film 2400 interposed therebetween define a disk-shaped space.

The top seal 2200 is bonded to the upper surface of the flow channel cell 2300 to provide a seal. On top of this, the pipette port 2100 is disposed.

The pipette port 2100 has a fluid inlet channel 2130 at its center and a pipette receiving portion 2131 that receives the tip of a pipette and that continues to the fluid inlet channel 2130. The top seal 2200 has a flow channel inlet hole 2230 leading from the pipette port 2100 to the flow channel cell 2300.

The sensor chip support 2600 is pressed against the waste liquid tank 2800 with the O-ring 2700 provided therebetween. The flow channel cell 2300 located above has a fitting claw 2380 that fits into a claw receiver 2880 of the waste liquid tank 2800. Thus, the pipette port 2100, the top seal 2200, the flow channel cell 2300, the adhesive film 2400, the sensor chip 2500, and the sensor chip support 2600, which have been assembled by bonding, are firmly attached to the waste liquid tank 2800.

The fluid inlet channel 2130 of the pipette port 2100, the flow channel inlet hole 2230 of the top seal 2200, and the top opening of the fluid inlet 2330 of the flow channel cell 2300 are aligned with each other. Thus, a fluid introduced from a pipette (not illustrated) passes through the fluid inlet channel 2130 of the pipette port 2100, the flow channel inlet hole 2230 of the top seal 2200, and the fluid inlet 2330 of the flow channel cell 2300, and is then introduced into the disk-shaped space 2320.

The contact opening 2260 of the top seal 2200, the contact through-hole 2360 of the flow channel cell 2300, and the contact opening 2460 of the adhesive film 2400 are aligned so as to be disposed above the contact terminals 2560 of the sensor chip 2500. Thus, connection pins (not illustrated) can be electrically connected to the contact terminals 2560 of the sensor chip 2500 from the outside of the fluidic device 2000 through these openings and the through-hole. In this manner, a surface electrochemical measurement can be performed on the introduced liquid via the electrode section 2520 of the sensor chip 2500 to obtain an electrical signal.

The fluid (waste liquid) leaving the disk-shaped space 2320 of the flow channel cell 2300 flows up through the fluid outlet 2340 of the flow channel cell 2300, passes through a channel 2341 formed of a groove 2341 on the upper surface of the flow channel cell 2300 and the top seal 2200, and is guided downward through a second fluid outlet 2342. The waste liquid then passes through a through-hole 2440 in the adhesive film and a through-hole 2640 in the sensor chip support 2600, falls into a waste liquid storage space 2840 in the waste liquid tank 2800, and is collected in a sealed manner.

An air opening 2270 in the top seal 2200, an air through-hole 2370 in the flow channel cell 2300, an air opening 2470 in the adhesive film 2400, and an air through-hole 2670 in the sensor chip support 2600 are aligned with each other. That is, the waste liquid storage space 2840 of the waste liquid tank 2800 is fluidly connected to the outside of the fluidic device 2000 to form an air vent. Thus, when a waste liquid is introduced into the waste liquid storage space 2840, air inside the waste liquid storage space 2840 sealed by the O-ring 2700 can escape to the outside. In other words, the waste liquid can flow to the storage space 2840 in the waste liquid tank 2800.

### <Comparative Example: Straight-Type Flow Channel>

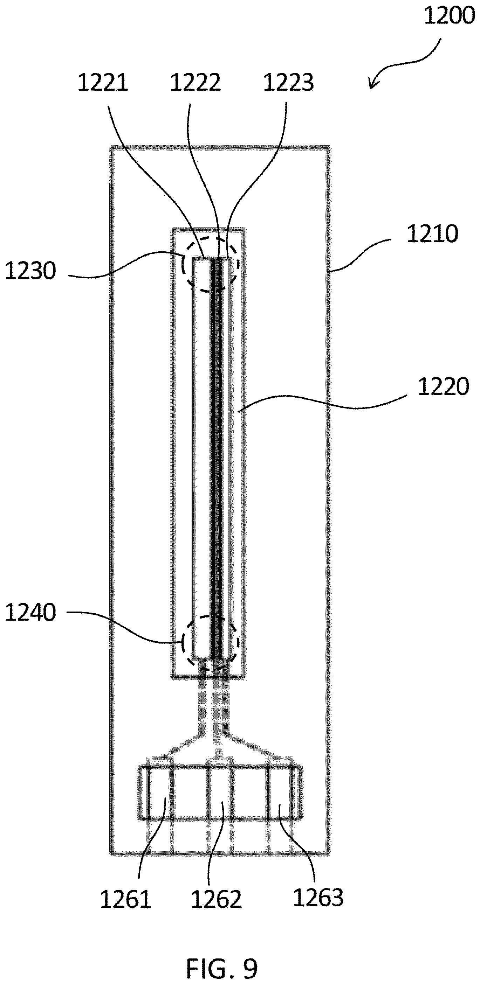

Fig. 9 is a top view of a fluidic device 1200 having a straight-type flow channel as a comparative example. However, the solid and dashed lines are not to be construed as corresponding to whether or not they are visible from the top, but are merely used to schematically illustrate the configuration of components in the fluidic device 1200.

The fluidic device 1200 has a straight (linear) space 1220 inside a main body 1210. A fluid inlet 1230 and a fluid outlet 1240 are disposed near the end portions of the straight space 1220. The fluid inlet 1230 and the fluid outlet 1240 are disposed perpendicular to the plane of the main body 1210 or the straight space 1220. A fluid is introduced into the straight space 1220 through the fluid inlet 1230, flows in the straight space 1220 in its longitudinal direction, fills the straight space 1220, and is guided to the outside through the fluid outlet 1240.

A sensing electrode including electrodes 1221, 1222, and 1223 extending in the longitudinal direction is disposed in the straight space 1220. In this comparative example, the sensing electrode includes three electrodes for electrochemical measurement. The sensing electrode includes a working electrode 1221, a reference electrode 1222, and a counter electrode 1223. These electrodes are connected to respective connection terminals 1261, 1262, and 1263 with lead wires provided therebetween. The connection terminals are exposed on the outer surface of the main body 1210 and can be electrically connected from the outside.

### <Comparison between Disk Type and Straight Type>

In general, it is recognized that in sensing devices employing a stopped-flow method, the sensing performance decreases when the measurement liquid has a low volume. One reason for this is presumably that, in the stopped-flow method, it is difficult to completely replace a measurement space filled with a preceding liquid with a measurement liquid, and it is difficult to remove trapped air bubbles, compared to a flow method, in which the measurement liquid is constantly supplied. In this experimental example, a disk-shaped flow channel and a straight-type flow channel were compared in terms of sensing performance with respect to the amount of liquid introduced, particularly at low volumes. The device configuration used is described below.

The disk-shaped flow channel had the same configuration as illustrated in Fig. 7. The shape of the disk-shaped space was 6.0 mm in diameter × 0.3 mm in height. Thus, the volume of the internal space was about 8.48 mm³ = 8.48 µL. Three electrodes having substantially the same configuration as illustrated in Fig. 7 were disposed on a base in the space.

The straight-type flow channel had an internal space of 20.5 mm in length × 3.0 mm in width × 0.3 mm in height. Thus, the volume of the internal space was about 18.45 mm³ = 18.45 µL. Three electrodes having substantially the same configuration as illustrated in Fig. 9 were disposed on a base in the space.

An enzyme membrane with fructosyl amino acid oxidase (FAOD) immobilized by cross-linking with bovine serum albumin (BSA) was formed on the electrodes.

A HEPES solution (10 mM HEPES, 150 mM NaCl, trace amount of preservative, pH 8.0) was prepared. A solution prepared by dissolving 14 to 15 µM fructosyllysine (FK) in the HEPES solution was provided as a measurement solution. Each flow channel was filled with the HEPES solution to condition the enzyme membrane for measurement. Thereafter, the measurement solution was introduced into each flow channel device in different amounts (50 to 1,000 µL) by pipetting, and a change in current output from the electrodes over time was measured. In the graph illustrated below, the current value after a predetermined time (about 120 seconds) from the introduction of the solution was used.

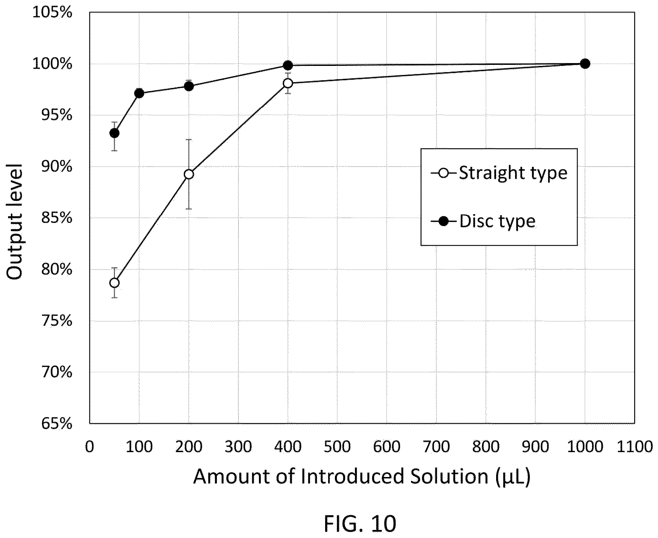

Fig. 10 illustrates the current value (vertical axis) obtained versus the amount of the solution (horizontal axis) for each device. In Fig. 10, the current values are normalized to 100% at 1,000 µL (1 mL).

In the straight-type flow channel, the output decreased significantly as the volume decreased, particularly at 400 µL or less. That is, at low volumes, a decrease in output was observed. In contrast, in the disk-shaped flow channel, a decrease in output was observed at 200 µL or less, but even at 50 µL, the decrease in output was only about 7%. In the disk-shaped flow channel, the problem of a significant power reduction at low capacities, observed in the straight-type flow channel, was not recognized. Thus, it was found that the disk-shaped flow channel had high sensing performance even for a low volume of a solution introduced.

The above results are discussed below as one interpretation. In the straight-type flow channel, the introduced measurement solution is considered to flow most easily through the center portion (center portion in the width direction) of the flow channel in terms of fluid dynamics. That is, when a sufficient amount of the solution is introduced (for example, 1,000 µL), the entire volume of the HEPES solution in the flow channel is considered to have been replaced by the measurement solution. However, when a sufficient amount of solution was not introduced (for example, 400 µL or less), it is considered that the entire volume of the HEPES solution in the flow channel was not replaced by the measurement solution. Thus, the measurement solution came into contact with only a portion of the total area of the electrodes. This is believed to have led to a decrease in the measured current. This situation is considered to worsen as the amount introduced decreases, which is consistent with the tendency of the experimental results illustrated in Fig. 10. In other words, the straight-type flow channel has a low solution conversion efficiency, whereas the disk-shaped flow channel has a high conversion efficiency.

A low solution conversion efficiency indicates that the preceding solution remains. This is a phenomenon that occurs when two different fluids come into contact with each other, and can cause variations in solution conversion efficiency, i.e., the sensing output. In fact, the inventors have observed such variations in the straight-type flow channel. In contrast, the disk-shaped flow channel has high conversion efficiency and is less likely to cause variations in output while allowing a certain amount of the preceding solution to remain.

Thus, the disk-shaped flow channel has a high solution conversion efficiency for a low volume of the measurement solution introduced, enabling highly sensitive and/or highly stable measurements.

The above discussion is an example, and there are other possible interpretations of the experimental results. This disclosure is not exclusive of other scientific and technical interpretations, considerations, theories, and so forth.

### <Effect of Height of Disk-Shaped Space>

In the present example, the effect of the height of the disk-shaped flow channel on the output current characteristics was examined. Flow channel devices having internal spaces with a diameter of 6.0 mm and heights of 0.1 mm, 0.28 mm, 0.6 mm and 1.0 mm were prepared. Three electrodes having substantially the same configuration as illustrated in Fig. 7 were disposed on a base in the space.

An enzyme membrane with fructosyl amino acid oxidase (FAOD) immobilized by cross-linking with bovine serum albumin (BSA) was formed on the electrodes. The enzyme membrane had a thickness of about 25 µm, which was smaller than the accuracy of the height of the internal space. In this way, the four types of flow channel devices had the same base shape and area, the same enzyme membrane, and different internal space heights.

A HEPES solution was prepared in the same manner as in the experiment illustrated in Fig. 10. A solution obtained by dissolving 20 µM FK as a substrate in the HEPES solution as a solvent was prepared as a substrate solution. Each flow channel was filled with the HEPES solution to condition the enzyme membrane for measurement. Thereafter, 100 µL of the substrate solution was introduced into each flow channel device by pipetting, and a change in current output from the electrodes over time was measured.

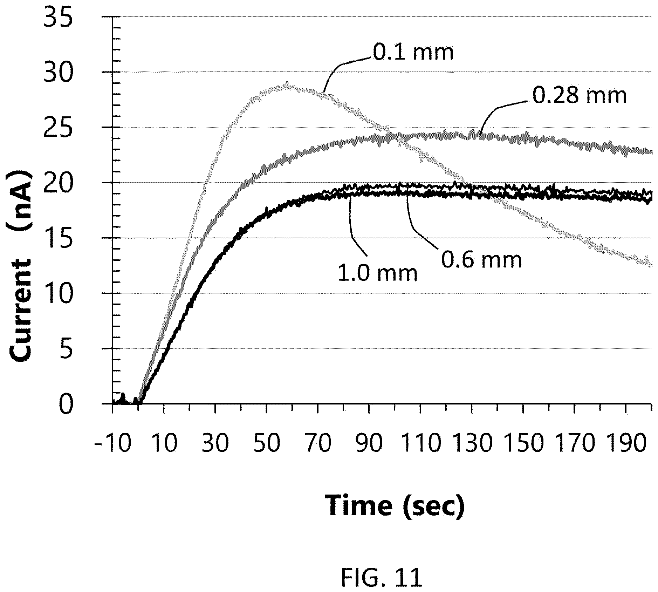

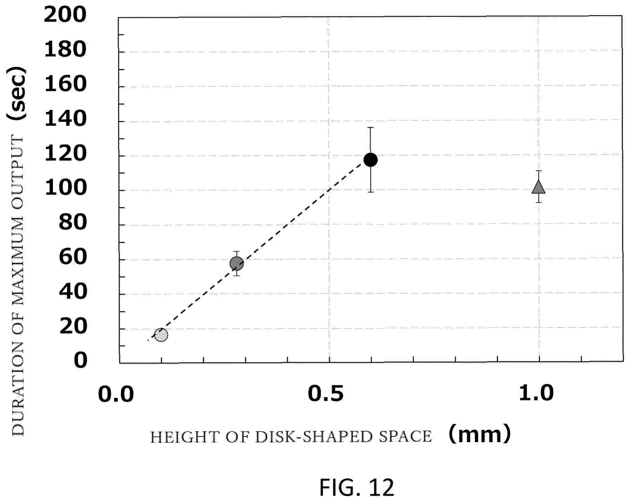

Fig. 11 illustrates the change in output current over time from the introduction of the substrate solution (time t = 0) obtained for each flow channel device. The multiple flow channel devices were prepared at each height, and measurements were performed. However, Fig. 11 illustrates one example for each. The device with a height of 0.1 mm exhibited the highest maximum output (about 28 nA) and the fastest time to reach the maximum output (about 58 seconds), but the maximum output was maintained for only a short period of time. The device with a height of 0.28 mm exhibited a lower maximum output (about 24 nA) and a more delayed time to reach the maximum output (about 120 seconds), but the time for which the maximum output was maintained was significantly long. Each of the devices with heights of 0.6 mm and 1.0 mm exhibited a lower maximum output and a more delayed time to the maximum output, but the time for which the maximum output was maintained was longer than that of the device with a height of 0.28 mm. Fig. 12 illustrates the relationship between the height of the disk-shaped space and the duration of maximum output.

The device with a height of 0.6 mm illustrated in Fig. 11 exhibited only a slightly lower maximum output current than that of the device with a height of 1.0 mm. However, no substantial difference was observed between the two. That is, it is presumed that there is substantially no change in the characteristics at a height of 0.6 mm or more (see Fig. 12). Of course, if the height is markedly large, the trends of these characteristics may change. For example, the height may be 1 mm or less. The measurement volume can be small.
In addition, the inclusion of air bubbles can be inhibited.

Logically, the device with a height of 0.1 mm, which exhibited the highest maximum output current, is considered to have the highest measurement sensitivity. However, the maximum output currents exhibited by the devices with heights of 0.6 mm and 1.0 mm provide sufficient measurement sensitivity for the devices of the present disclosure. The time of the maximum output current varies depending on variations in the timing of introduction of the substrate solution into the flow channel device, the solution feeding speed, and so forth. That is, a longer time for which the maximum output current is maintained is advantageous for the stability of the measurement results. For example, the introduction of the substrate solution into the flow channel device can be the starting point for the calculation of the measurement time. For example, a current value after a certain period of time from the measurement time when the substrate solution is introduced into the flow channel device may be used as the measured value. Fig. 12 illustrates the relationship between the height of the flow channel device and the duration of maximum output. The time from the time when the maximum output current was given to the time when the current value decreased by 1.2 nA, which is the maximum value of the variation of the current data, was calculated as the duration of maximum output.

The device with a height of 0.6 mm and the device with a height of 1.0 mm provided a duration of maximum output of about 100 seconds. In fact, the maximum output current is stable; hence, the error in this calculated duration is large. As mentioned above, in the present example, there was a slight difference between the device with a height of 0.6 mm and the device with a height of 1.0 mm. However, it is found that they generally exhibit the same characteristics. Therefore, as mentioned above, it is presumed that at a height of 0.6 mm or more, there is substantially no change in the duration of maximum output (Fig. 12).

The duration of maximum output increased almost linearly and monotonically from a height of 0.1 mm to 0.6 mm (Fig. 12). For example, if this time is one minute (60 seconds), the measurement can be performed stably. At a height of 0.28 mm, the duration of maximum output is about 60 seconds. This is considered to be substantially sufficient. Thus, in some embodiments, the height of the internal space may be 0.28 mm or more. At a height of 0.3 mm, the duration of maximum output of about 60 seconds can be obtained. Thus, in some embodiments, the height of the internal space may be 0.3 mm or more. The height of the internal space may be a value, such as 0.4 mm, 0.5 mm, or greater.

In the present example, the thickness of the enzyme membrane was about 25 µm as described above. The inventors have confirmed a similar tendency even at 10 µm to 35 µm (not illustrated). When the thickness of the enzyme membrane was less than 10 µm, the output current value changed abruptly, and its stability was low. When the thickness of the enzyme membrane was 40 µm, the diffusion of the substrate in the enzyme membrane was slow, and thus the current measurement was inefficient.

A short duration of maximum output often indicates that the current value decreases abruptly after the time of the maximum output current. Variations in the characteristics of the enzyme membrane occur to a certain extent due to the production process. A configuration that causes an abrupt change in current can be a factor in reducing the reproducibility of the measurement. Therefore, it is meaningful to have a certain degree of the duration of maximum output, at least for high reproducibility. A long duration of maximum output indicates that the diffusion/supply rate of the substrate to the enzyme membrane or the concentration gradient of the substrate in the enzyme membrane is constant for a long time. Therefore, high reproducibility can be obtained. In addition, the accuracy of the measurement value of the GA value obtained based on the output current curve is also improved.

There are various methods for calculating the GA value from the output current characteristic (current change over time/also called curve, waveform data, etc.). For example, a current value at a predetermined time from the time the substrate solution is introduced into the device space may be acquired. For example, multiple current values on the current curve may be selected or acquired. For example, a statistical value may be determined from the current curve. For example, a certain statistical value may be determined from current values at a plurality of times. The GA value may be calculated based on one or more statistical values. For example, the GA value may be determined based on the maximum output current value and the current values 10 seconds before and after the maximum output current value. For example, the GA value may be determined based on the area provided by the output current curve. These are merely examples, and other calculation methods can also be used.

The present disclosure includes the following embodiments.
A001 A fluidic device, including:
   a device main body having a disk-shaped space for containing a fluid;
   a fluid inlet configured to introduce the fluid tangentially into the disk-shaped space at the 0 o'clock position of the substantially circumferential portion of the disk-shaped space; and
   a fluid outlet configured to guide the fluid out of the disk-shaped space at the 6 o'clock to 12 o'clock position of the substantially circumferential portion of the space.
A011 The fluidic device described in embodiment A001,
   in which the fluid outlet is configured to guide the fluid out of the disk-shaped space at the 7 o'clock to 12 o'clock position of the substantially circumferential portion of the space.
A012 The fluidic device described in embodiment A011,
   in which the fluid outlet is configured to guide the fluid out of the disk-shaped space at the 9 o'clock to 12 o'clock position of the substantially circumferential portion of the space.
A013 The fluidic device described in any one of embodiments A001 to A012,
   in which the fluid outlet is disposed in the disk-shaped space at a position in a counterclockwise direction from the inlet (on the upstream side of the flow of the fluid).
A021 The fluidic device described in any one of embodiments A001 to A013,
   in which the disk-shaped space has a volume of 1 µL to 100 µL.
A022 The fluidic device described in embodiment A021,
   in which the disk-shaped space has a volume of 3 µL to 20 µL.
A023 The fluidic device described in embodiment A021,
   in which the disk-shaped space has a height of 0.28 mm, 0.3 mm, 0.4 mm, 0.5 mm, or 0.6 mm, or greater.
A025 The fluidic device described in any one of embodiments A001 to A023,
   in which the total of the volume of the disk-shaped space and the volume of the fluid inlet is 2 µL to 200 µL.
A026 The fluidic device described in embodiment A025,
   in which the total of the volume of the disk-shaped space and the volume of the fluid inlet is 6 µL to 40 µL.
A027 The fluidic device described in any one of embodiments A001 to A026,
   in which the volume of the fluid inlet is substantially 50%, 100%, 150%, or 200% of a volume of the disk-shaped space.
A031 The fluidic device described in any one of embodiments A001 to A027 further includes:
   a sensor at the inner wall of the disk-shaped space.
A032 The fluidic device described in embodiment A031,
   in which the sensor is a biosensor.
A033 The fluidic device described in embodiment A031 or A032,
   in which the sensor includes an electrode.
A034 The fluidic device described in embodiment A033,
   in which the electrode includes a hydrogen peroxide electrode.
A035 The fluidic device described in embodiment A034,
   in which the sensor further includes an enzyme membrane over the hydrogen peroxide electrode.
A036 The fluidic device described in embodiment A035,
   in which the enzyme membrane contains an oxidase.
A037 The fluidic device described in embodiment A036,
   in which the oxidase is FAOD.
A038 The fluidic device described in any one of embodiments A035 to A037,
   in which the enzyme membrane contains a protease.
A039 The fluidic device described in embodiment A036,
   in which the oxidase is a glucose oxidase.
A041 The fluidic device described in any one of embodiments A031 to A039,
   in which the sensor is a protein sensor.
A042 The fluidic device described in embodiment A041,
   in which the sensor includes a glycoalbumin sensor and/or an albumin sensor.
A051 The fluidic device described in any one of embodiments A031 to A042,
   in which the fluidic device is configured to include an optical sensor outside the disk-shaped space or is configured to be combined with an optical sensor outside the disk-shaped space.
A061 The fluidic device described in any one of embodiments A001 to A051,
   in which the fluidic device is a stopped-flow fluidic device.

While several embodiments and examples of the present disclosure have been described above, these embodiments and examples illustrate the present disclosure. For example, each of the embodiments described above has been described in detail in order to explain the present disclosure in an easy-to-understand manner, and dimensions, configurations, materials, and circuits may be additionally changed as necessary. Embodiments in which one or more features of the present disclosure described above are arbitrarily combined are also included in the scope of the present disclosure. It is intended that the appended claims cover numerous modifications to the embodiments without departing from the spirit and scope of the present disclosure. Accordingly, the embodiments and examples disclosed herein have been shown by way of examples and should not be considered as limiting the scope of the present disclosure.

## Claims

1. A fluidic device, comprising:
a device main body having a disk-shaped space for containing a fluid;
a fluid inlet configured to introduce the fluid tangentially into the disk-shaped space at a 0 o'clock position of a substantially circumferential portion of the disk-shaped space; and
a fluid outlet configured to guide the fluid out of the disk-shaped space at a 6 o'clock to 12 o'clock position of the substantially circumferential portion of the space.

2. The fluidic device according to Claim 1,
wherein the fluid outlet is configured to guide the fluid out of the disk-shaped space at a 7 o'clock to 12 o'clock position of the substantially circumferential portion of the space.

3. The fluidic device according to Claim 2,
wherein the fluid outlet is configured to guide the fluid out of the disk-shaped space at a 9 o'clock to 12 o'clock position of the substantially circumferential portion of the space.

4. The fluidic device according to Claim 1,
wherein the fluid outlet is disposed in the disk-shaped space at a position in a counterclockwise direction from the inlet (on an upstream side of a flow of the fluid).

5. The fluidic device according to Claim 1,
wherein the disk-shaped space has a volume of 1 µL to 100 µL.

6. The fluidic device according to Claim 5,
wherein the disk-shaped space has a height of 0.3 mm or more.

7. The fluidic device according to Claim 1,
wherein a total of a volume of the disk-shaped space and a volume of the fluid inlet is 2 µL to 200 µL.

8. The fluidic device according to Claim 1,
wherein a volume of the fluid inlet is substantially 50%, 100%, 150%, or 200% of a volume of the disk-shaped space.

9. The fluidic device according to Claim 1, further comprising:
a sensor at an inner wall of the disk-shaped space.

10. The fluidic device according to Claim 9,
wherein the sensor is a biosensor.

11. The fluidic device according to Claim 10,
wherein the electrode includes a hydrogen peroxide electrode.

12. The fluidic device according to Claim 11,
wherein the sensor further includes an enzyme membrane over the hydrogen peroxide electrode.

13. The fluidic device according to Claim 12,
wherein the enzyme membrane contains FAOD and a protease.

14. The fluidic device according to Claim 12 or 13,
wherein the enzyme membrane contains a glucose oxidase.

15. The fluidic device according to Claim 10,
wherein the sensor includes a glycoalbumin sensor and/or an albumin sensor.

16. The fluidic device according to Claim 1,
wherein the fluidic device is configured to include an optical sensor outside the disk-shaped space or is configured to be combined with an optical sensor outside the disk-shaped space.

17. The fluidic device according to Claim 1,
wherein the fluidic device is a stopped-flow fluidic device.
